Europäisches Patentamt

European Patent Office

Office européen des brevets

(19)

(11) Numéro de publication: **0 233 816
B1**

(12) **FASCICULE DE BREVET EUROPEEN**

(45) Date de publication du fascicule du brevet: **05.09.90**

(21) Numéro de dépôt: **87400202.5**

(22) Date de dépôt: **29.01.87**

(51) Int. Cl.⁵: **C 07 C 59/105,** C 07 C 51/31, C 07 C 51/235

(54) Procédé d'oxydation d'aldoses et un catalyseur mis en oeuvre.

(30) Priorité: **30.01.86 FR 8601306**

(43) Date de publication de la demande:
**26.08.87 Bulletin 87/35**

(45) Mention de la délivrance du brevet:
**05.09.90 Bulletin 90/36**

(84) Etats contractants désignés:
**AT BE DE ES GB IT NL SE**

(56) Documents cités:
**EP-A-0 109 259
EP-A-0 112 261
EP-A-0 142 725**

(73) Titulaire: **Roquette Frères
F-62136 Lestrem (FR)**

(72) Inventeur: **Fuertes, Patrick
207, rue de Paris
F-59000 Lille (FR)**
Inventeur: **Flèche, Guy
49 rue G. Charlet "Le Sart"
F-59600 Merville (FR)**

(74) Mandataire: **Koch, Gustave et al
Cabinet PLASSERAUD 84, rue d'Amsterdam
F-75009 Paris (FR)**

Le dossier contient des informations techniques présentées postérieurement au dépôt de la demande et ne figurant pas dans le présent fascicule.

Courier Press, Leamington Spa, England.

EP 0 233 816 B1

**Description**

La présente invention a essentiellement pour objet un procédé d'oxydation d'aldoses en acides aldoniques.

Elle vise tout particulièrement l'application du susdit procédé à la préparation de l'acide gluconique par oxydation du glucose.

Bien que, du point de vue industriel, l'acide gluconique soit actuellement préparé par fermentation, il a déjà été proposé de le préparer par oxydation chimique, électrolytique et catalytique en présence de catalyseurs à base de métaux nobles pour tenter de remédier notamment aux inconvénients des procédés de fermentation qui sont liés notamment à la complexité ds infrastructures nécessaires à leur mise en oeuvre, aux risques de pollution microbiologique, aux durées de réaction élevées.

Ainsi,

en 1947, K. HEYNS (Annales 558, pages 187 à 192) décrit l'oxydation des monsaccharides et en particulier du glucose à l'aide d'une catalyseur obtenu par dépôt de platine sur charbon, et

en 1955, le brevet GB 786 288 décrit la préparation de gluconate de sodium à l'aide d'un catalyseur obtenue par dépôt de palladium sur charbon.

Le principal obstacle au développment de cés procédés d'oxydation catalytique tient au fait que les catalyseurs à base de platine ou de palladium sont d'une sélectivité médiocre. En effet, ces catalyseurs ont l'inconvénient d'oxyder simultanément les groupements aldéhydes et les fonctions alcool, ce qui génère dans le cas particulier de l'oxydation du glucose en acide gluconique, l'apparition de produits indésirables tels que l'acide glucarique. Ces réactions de suroxydation incontrôlées sont d'autant plus marquées que les taux de conversion du glucose est élevé, si bien qu'il et difficile d'obtenir un rendement en acide gluconique supérieur à 92—94%.

D'autre part, les catalyseurs à base de palladium ou de platine ne permettent pas, malgré le caractère alcalin du milieu au sein duquel ils sont mis en oeuvre, de maintenir au cours de la réaction d'oxydation une cinétique suffisamment élevée pour éviter l'isomérisation du glucose en fructose et de fait l'oxydation subséquente du fructose en acide 2-céto-gluconique.

our améliorer la sélectivité et la vitesse de cess réactions d'oxydation, il a été proposé (brevet japonais JP 59—205 343) de constituer les catalyseurs mis en oeuvre par imprégnation préalable d'une sel de plomb sur un support constitué de charbon finement divisé suivi d'un dépôt de palladium, le plomb pouvant être remplacé par le sélénium (brevet JP 60—92240), ou encore par le bismuth (brevet EP 142 725).

Ces catalyseurs apportent une certaine amélioration au niveau de la sélectivité de la réaction d'oxydation, mais ne permettent pas d'assurer une production économique d'acides aldoniques de pureté constante, du fait qu'ils ne présentent pas une stabilité suffisante pour supporter les opérations de recyclage, critère essentiel pour que ces procédés d'oxydation catalytique soient économiquement viables, cette instabilité générant par ailleurs une diminution rapide de la pureté des produits ainsi obtenus.

L'invention a donc pour but, surtout, de remédier à ces inconvénients et de fournir un procédé de préparation d'acides aldoniques et notamment de l'acide gluconique répondant mieux que ceux qui existent déjà aux divers desiderata de la pratique.

Or, la Société Demanderesse a le mérite d'avoir pu démontrer que, de façon surprenante et inattendue, une production économique d'acides aldoniques et notamment d'acide gluconique de pureté constante devenait possible dès lors que, dans un procédé du genre en question, on a recours à une catalyseur obtenu par apport d'une quantité efficace d'au moins des métaux du groupe de ceux constituant les classes IV, V et VI, ou promoteur, sur un catalyseur constitué par du palladium sur support inerte.

Il s'ensuit que le procédé de préparation d'acides aldoniques et notamment d'acide gluconique conforme à l'invention est caractérisé par le fait que l'on oxyde un aldose et notamment du glucose en milieu alcalin et au moyen d'un gaz contenant de l'oxygène, en ayant recours à un catalyseur obtenue par apport d'une quantité efficace d'au moins un des métaux du groupe de ceux constituant les classes IV, V et VI, ou promoteur, sur un catalyseur constitué par du palladium sur support inerte.

Outre ce procédé de préparation des acides al'doniques, l'invention vise les susdits catalyseurs dans leur application audit procédé.

Les aldoses autres que le glucose, pouvant être oxydés par le procédé conforme à l'invention, sont ceux du groupe comprenant l'érythrose, le thréose, le ribose, l'arabinose, le xylose, le lyxose, l'allose, l'altrose, le gulose, le mannose, l'idose, le galactose et le talose.

Ces aldoses peuvent se présenter sous la forme de corps purs cristallises ou non ou sous la forme de solutions technqieus, c'est-à-dire de moindure pureté.

A titre d'exemple, dans le cas du glucose, on sait que celui-ci peut être avantageusement obtenu sous forme de solutions par des procédés d'hydrolyse enzymatique et/ou acide. Ces solutions communément appelées hydrolysat d'amidon sont définies par leur pouvoir réducteur ou DE (Detrose-Equivalent).

Le procédé selon l'invention peut être mis en oeuvre sur tout hydrolysat dont le DE est supérieur à 90, de préférence supérieur à 95.

Les acides aldoniques, résultats de l'oxydation, sont des monoacides carboxyliques polyhdyroxylés dans lesquels la fonction aldéhyde des aldoses est oxydée en groupement carboxylique.

Les "Promoteurs" préférés sont le bismuth, le plomb, l'antimoine, l'étain ou le sélénium, avec une

2

EP 0 233 816 B1

préférence toute particulière pour le bismuth et le plomb. Ils sont préférentiellement choisis sous forme de sels, afin que leur solubilisation, en milieu aqueux, généralement acide, soit facilitée.

L'apport des promoteurs sur le catalyseur constitué par du palladium sur support inerte peut se faire par imprégnation.

Dans ce but, on mélange la solution de promoteur à une suspension aqueuse du catalyseur supporté à base de palladium; l'imprégnation est obtenue en maintenant sous agitation le mélange pendant une durée d'au moins quelques secondes à quelques heures. Ce laps de temps est directment dépendant de la cinétique de l'étape d'imprégnation. Ils est généralement compris entre 15 minutes et 2 heures.

La suspension de catalyseur, supporté à base de palladium, ainsi imprégnée, est alors rendue alcaline par ajout d'une base telle que la soude, la potasse ou le carbonate de sodium. Cette opération précède l'étape de réduction du promoteur qui est effectuée à une température comprise entre 20 et 100°C à l'aide d'agents chimiques réducteurs du type formol, formiate de sodium, borohydrure de sodium, acid hypophosphoreux, hydrazine, glucose ou autres sucres réducteurs.

Le catalyseur ainsi réduit est filtré, lavé et employé tel quel.

Les catalyseurs préférés dans le cadre de la présente invention sont ceux obtenus par dépôt de bismuth et/ou de plomb sur un catalyseur à base de palladium supporté sur charbon.

La teneur du catalyseur en palladium exprimée en tant que métal est généralement comprise entre 1 et 10% en poids par rapport au support inerte.

La quantité efficace de promoteur, notamment de bismuth et/ou de plomb, exprimée en tant que métal, est comprise entre 1 et 300% et, de préférence, entre 5 et 100% en poids par rapport au palladium.

Pour la mise en oeuvre du procédé selon l'invention

on introduit dans une réacteur muni dispositif d'agitation, une solution aqueuse d'un aldose, notamment de glucose, ou d'un mélange d'aldoses, la concentration en aldose étant de préférence comprise entre 5 et 60% en poids, la limite inférieure étant seulement dictée par un souci de rentabilité du procédé et la limite supérieure tenant compte de la solubilité de l'oxygène dans les milieux très visqueux et du risque de cristallisation du sel de l'acide aldonique formé pendant la réaction.

on disperse dans cette solution une quantité du catalyseur mis en oeuvre conformément à l'invention telle quye la quantité de palladium exprimée en tant que métal soit comprise entre 0,005 et 1% et, de préférence, entre 0,01 et 0,4% en poids par rapport à la quantité d'aldose,

on amorce la réaction d'oxydation par l'apport simulané d'un balayage d'air ou d'un gaz contenant de l'oxygène et d'un agent alcalin, la température de réaction se situant généralement entre 20 et 90°C, de préférence entre 25 et 60°C, pour un temps de réaction compris entre 30 minutes et 5 heures.

A titre d'exemple, on signale que l'oxydation du glucose en gluconate de sodium est effectuée préférentiellement à une concentration de la solution de glucose comprise entre 20 et 40% en poids.

L'argent alcalin utilisé qui choisi parmi l'hydroxyde de sodium, l'hydroxyde de potassium, l'hydroxyde de calcium, l'hydroxyde de lithium, l'hydroxyde de magnésium, en fonction du but visé. Ainsi, on utilisera de la soude pour obtenir le sel de sodium de l'acide aldonique correspondant à l'aldose mis en oeuvre; on peut également utiliser du carbonate de zinc ou de manganèse ou tout autre sel de zinc ou de manganèse pour lesquels le hydroxydes correspondants sont obtenus in situ par addition d'un agent alcalin tel que l'hydroxyde de sodium ou l'hydroxyde de potassium.

L'agent alcalin est également destiné à neutraliser l'acide aldonique formé afin de maintenir une activité catalytique constante au cours de la réaction; il doit permettre de maintenir le pH du milieu réactionnel à une valeur suffisante pour assurer la désorption de l'acide aldonique formé et éviter la suroxydation de celui-ci sans que pour autant cette valeur soit trop élevée, une valeur trop élevée pouvant donner naissance à des réactions d'isoméristion d'aldose en cétose.

Dans la pratique, le pH est maintenu à une valeur comprise entre 7,5 et 11,0 de préférence entre 8,0 et 10,0.

Le procédé conforme à l'invention permet d'obtenir un taux de conversion des aldoses employés supérieur à 95% et plus particulièrement compris entre 98 et 100% avec une sélectivité élevée de l'ordre de 97 à 100%.

Ces performances remarquables sont d'autant plus exceptionnelles qu'elles ne sont pas altérées par un nombre important de recyclage des catalyseurs mis en oeuvre conformément à l'invention, qui présentent une grande stabilité et dont la durée de vie est nettement supérieure à celle des catalyseurs antérieurement utilisés et obtenus par imprégnation d'un promoteur avant le dépôt de métal noble, les catalyseurs utilisés conformément à l'invention pouvant de plus être facilement régénérés par dépôt d'une nouvelle charge de promoteur.

Les acides aldoniques peuvent être avantageusement employés comme agents chélatants, comme agents destinés au lavage des articles en verre et en métaux, ou comme additifs pour détergents, médicaments ou additifs alimentaires.

Ainsi, le gluconate de sodium est utilisé comme fluidifiant-réducteur d'eau, ou retardateur de prise dans les liants hydrauliques.

L'inventon pourra être mieux comprise à l'aide des exemples non limitatifs qui suivent et qui illustrent entre autres des modes de realisation avantageux de l'invention.

On indique tout d'abord la préparation du catalyseur mis en oeuvre conformément à l'invention (Exemple 1) et la préparation d'une catalyseur utilisé dans l'art antérieur (Exemple 2).

3

# EP 0 233 816 B1

## Exemple 1

Préparation d'un catalyseur à 5% de Pd et 3,5% de Bi sur charbon par dépôt de bismuth sur un catalyseur palladium sur charbon ou Pd/C du commerce.

Une quantité de 6 g de catalyseur sec Pd/C du commerce (DEGUSSA 198 R/W à 5% Pd) est mise en suspension dans 80 ml d'eau distillée acidifiée par 1 ml d'acide chlorhydrique concentré (37% HCl). A cette suspension, on ajoute une solution constituée de 0,3 g de subnitrate de bismuth dissous dans un mélange de 2 ml d'acide chlorhydrique concentré et de 5 ml d'eau distillée.

On établit une agitation pendant heures, puis on introduit 4 g de soude cauistique en solution dans 30 ml d'eau. Le mélange est porté à une température de 40—50°C pendant 4 heures, puis on ajoute 1,5 ml de formol (solution aqueuse à 37—38%). On porte le mélange à 85°C pendant 1 heure. Le catalyseur ainsi obtenu est filtré et lavé.

## Exemple 2

Préparation d'un catalyseur à 5% de Pd et 3,5% de Bi sur charbon par dépôt de bismuth sur un catalyseur palladium.

Une quantité de 6 g de charbon actif sec est mise en suspension dans 80 ml d'eau distillée. A cette suspension, on ajoute une solution constituée de 0,3 g de subnitrate de bismuth dissous dans un mélange de 3 ml d'acide chlorhydrique concentré et de 5 ml d'eau distillée.

On établit une agitation pendant 6 heures afin de laisser le bismuth s'adsorber complètement sur le charbon actif. On ajoute ensuite une solution de 0,5 g de chlorure de palladium 0,3 g de palladium métal) dans 1,5 ml d'acide chlorhydrique et 5 ml d'eau distillée. On ajoute 4 g de soude caustique en solution dans 30 ml d'eau et on porte le mélange à 40°C pendant 5 heures. Aprés addition de 1,5 ml d'une solution de formol à 37%, on maintient la suspension à 85°C pendant 1 heure. Le catalyseur ainsi obtenu est filtré et lavé.

Dans le cadre de l'exemple 3, on compare la stabilité du catalyseur mis en oeuvre conformément à l'invention à celle du catalyseur utilisé dans l'art antérieur.

## Exemple 3

On réalise une série d'expériences d'oxydation du glucose en introduisant chaque fois individuellement dans une enceinte réactionnelle d'une capacité de 1 litre, équipée d'un agitateur et d'un thermomètre, d'une tige frittée d'insufflation d'air, d'une électrode et d'un dispositif d'introduction en continu, une quantité de 666 g d'une solution aqueuse de glucose à 30% de matières sèches (contenant 200 g de glucose) ainsi qu'une quantité de 6 g de respectivement chacun des catalyseurs secs selon les exemples 1 et 2.

On fait réagir à 35°C et on insuffle de l'air tout en introduisant simultanément une solution aqueuse de soue à 30% de façon à maintenir le pH à une valeur e 8,8 ± 0,3.

On arrête la réaction lorsque la quantité théorique de soude a été consommée, ce qui donne la vitesse ou la durée de la réaction; le produit réactionnel est ensuite séparé par filtration et on détermine, d'une part, le pourcentage de produit recherché présent, ce qui donne la sélectivité de la réaction et, d'autre part, le pourcentage de sucres réducteurs résiduels.

On recycle sans régénération intemédiaire 40 fois, c'est-à-dire on procède à 40 expériences successives avec le catalyseur mis en oeuvre coformément à l'invention (exemple 1); on recycle 25 fois sans régénération dans le cas du catalyseur utilisé dans l'art antérieur (exemple 2).

Les résultlats réunis dans le tableau I sont ceux des respectivement 40 et 25 expériences successives.

TABLEAU I

| Nombre de Recy-clages | Catalyseur Pd/Bi/C (Bi déposé après Pd) Exemple 1 | | | Catalyseur Pd/Bi/C (Bi déposé après Pd) Exemple 1 | | |
|---|---|---|---|---|---|---|
| | Durée de la réaction | Sucres réducteurs (en %) | Rendement en gluconate (en %) | Durée de la réaction | Sucres réducteurs (en %) | Rendement en gluconate (en %) |
| 1 | 3 h 20 | 1,3 | 98,5 | 3 h 25 | 1,1 | 98,3 |
| 5 | 4 h 20 | 1,0 | 8,5 | 4 h 10 | 1,0 | 98,8 |
| 10 | 4 h 00 | 1,2 | 98,6 | 3 h 45 | 1,2 | 98,7 |
| 15 | 3 h 20 | 1,1 | 98,4 | 4 h 55 | 1,8 | 98,0 |
| 20 | 3 h 30 | 1,4 | 98,4 | 6 h 20 | 2,3 | 97,0 |
| 25 | 3 h 45 | 1,4 | 98,2 | 8 h 00 | 2,9 | 97,4 |
| 30 | 3 h 25 | 1,5 | 98,3 | | | |
| 35 | 3 h 40 | 1,9 | 98,0 | | | |
| 40 | 4 h 00 | 2,1 | 97,6 | | | |

Les résultats réunis dans le tableau I montrent que le catalyseur mis en oeuvre conformément à la présente invention (exemple 1) conserve une excellente activité catalytique; on n'observe pratiquement aucune augmentation de la durée de réaction pendant 40 essais et le rendement en gluconate de sodium ainsi que la teneur en sucres réducteurs restent également constants.

Le catalyseur utilisé dans l'art antérieur (exemple 2) montre une perte d'activité significative après 20 esais, avec augmentation du temps de réaction et des sucres réducteurs, le temps de réaction devenant rédhibitoire après 25 essais.

Dans l'exemple 4, on étudie l'influence, dans le catalyseur mis en oeuvre conformément à l'invention, de la proportion di bismuth.

### Exemple 4

a) Préparation du catalyseur Pd/Charbon.

Une quantité de 6 g de charbon actif est mise en suspension dans 100 ml d'eau distillée contenant 4 g de soude caustique. On ajoute à la suspension 0,5 g de chlorure de palladium (0.3 g de palladium exprimé en tant que métal) solubilisé dans 5 ml d'eau distillée additionnée de 1,5 ml d'acide chlorhydrique concentré. On laisse adsorber le palladium pendant 4 heures à 40—50°C, puis on ajoute 1,5 ml de formol (solution aqueuse à 37%) et on porte à 85°C pendant 1 heure.

Le catalyseur filtré est lavé avant dépôt du promoteur.

b) Dépôt de quantitiés décroisantes de promoteur (bismuth).

Le dépôt de bismuth sur le catalyseur Pd/C préparé comme indiqué ci-dessus est effectué de la même façon que dans l'exemple 1. On utilise successivement des solutions de subnitrate de bismuth contenant respectivement 0,6 g, 0,3, g, 0,15 g, 0,080 g, 0,040 g, ce qui conduit à autant de catalyseurs à respectivement 140%, 70%, 35%, 19% et 9% e bismuth exprimé en tant que métal par rapport au palladium exprimé en tant que métal.

On utilise le mode opératoire décrit à l'exemple 3 pour oxyder du glucose en acide gluconique.

Dans le tableau II, on a réuni:

la durée de la réaction,

la proportion de sucres réducteurs dans le produit obtenu,

la proportion de gluconate de Na et,

la proportion d'acide glucarique présent pour les réactions obtenues avec, d'une part, les cinq catalyseurs dont il vient d'être indiqué la concentration en bismuth et, d'autre part, avec le catalyseur Pd sur charbon actif sans promoteur (témoin).

TABLEAU II

| Teneur en bismuth par rapport au palladium (%) | Durée de la réaction | Sucres réducteurs | Acide glucarique | Gluconate de sodium |
|---|---|---|---|---|
| 140 | 4 h 20 | 1,4 | 0,8 | 98,2 |
| 70 | 3 h 10 | 1,3 | 1,0 | 97,7 |
| 35 | 3 h 10 | 1,2 | 1,2 | 97,8 |
| 19 | 2 h 40 | 1,8 | 1,3 | 97,6 |
| 9 | 4 h 10 | 2,6 | 1,7 | 96,5 |
| 0 (témoin) | 5 h 20 | 7,2 | 2,1 | 93,8 |

Les résultats obtenus montent que la teneur en bismuth n'a qu'une influence limitée sur la vitesse et la sélectivité de la réaction.

Dans l'exemple 5, on étudie l'influence de l'utilisation du Pb et de l'antimoine en tant que promoteur.

### Exemple 5

a) Préparation du catalyseur contenant du plomb comme promoteur.

Il s'agit d'un catalyseur à 5% de Pd et 2,5% de Pb sur charbon par dépôt de plomb sur un catalyseur Pd/C du commerce.

Une quantité de 6 g de catalyseur Pd/C sec du commerce (DEGUSSA 198 R/W à 5% Pd) est mise en suspension dans 80 ml d'eau distillée. On ajoute à cette suspension 20 ml d'une solution aqueuse contenant 0,3 g d'acétate de plomb. On laisse adsorber le plomb pendant 1 heure sous agitation. On ajoute 30 ml d'une solution aqueuse contenant 4 g de $Na_2CO_3$ et le mélange obtenu est porté à 40°C pendant 4 heures. On ajoute 1,5 ml de formol et on maintient la suspension à 85°C pendant 1 heure. Le catalyseur ainsi obtenue est ensuite filtré et lavé l'eau distillée.

b) Préparation du catalyseur contenant de l'antimoine comme promoteur.

Il s'agit d'un catalyseur à 5% de Pd et 1% e Sb sur charbon par dépôt d'antimoine sur un catalyseur Pd/C du commerce.

Une quantité de 6 g de catalyseur Pd/C sec du commerce (DEGUSSA 198 R/W à 5% Pd) est mise en suspension dans 80 ml d'eau distillée. On ajoute à cette suspension une solution aqueuse constituée de 0,1 g de $SbCl_3$, 2 ml de HCl concentré et 5 ml d'eau distillée. On maintient sous agitation pendant 3 heures et on ajoute 50 ml d'une soution aqueuse contenant 8 g de $Na_2CO_3$ puis goutte à goutte 10 ml d'une solution de soude à 0,1 N contenant 0,2 g de borohydrure de Na. Le mélange obtenue est porté à 85°C pendant 1 heure. Le catalyseur ainsi obtenu est ensuite filtré et lavé à l'eau distillée.

c) On reprend le mode opératoire de l'exemple 3 pour oxyder du glucose en gluconate de sodium en utilisant les deux susdits catalyseurs dopés et le catalyseur à base de palladium seul (témoin).

Les résultats sont réunis dans le tableau III.

TABLEAU III

| Catalyseur utilisé | Durée de la réaction | Sucres réducteurs (en %) | Rendement en gluconate (en %) |
|---|---|---|---|
| 5% Pd — 2,5% Pd | 2 h 10 | 3,0 | 96,0 |
| 5% Pd — 1,0% Pd | 2 h 40 | 3,1 | 96,1 |
| 5% Pd (témoin) | 5 h 20 | 7,2 | 93,8 |

Ces résultats montrent que les promoteurs utilisés augmentent considérablement la cinétique et la sélectivité de la réaction.

6

Dans l'exemple 6 qui suit, on aplique le procédé conforme à l'invention à l'oxydation du glucose et de trois aldoses autres que le glucose.

Le catalyseur utilisé est celui de l'exemple 1.

Le mode opératoire et celui de l'exemple 3, le glucose étant successivement remplacé par:

le D(+) mannose
le D(−) arabionose
le D(−) ribose.

La durée de la réaction et le rendement en acide aldonique correspondant sont réunis dans le tableau IV.

### TABLEAU IV

| Nature de l'aldose | Durée de la réaction | Tendement en acide aldonique correspondant (en %) |
|---|---|---|
| D(+) glucose | 3 h 20 | 98,0 |
| D(+) mannose | 2 h 30 | 95,7 |
| D(−) arabinose | 2 h 40 | 98,8 |
| D(−) ribose | 2 h 00 | 96,9 |

Les résultats obtenus sont comparables à ceuc obtenus avec le glucose.

Comme il va de soi et comme il résulte d'ailleurs déjà de ce qui précède, l'invention ne se limite nullement à ceux de ses modes d'application et de réalisation qui ont été plus spécialement envisagés; elle en embrasse, au contraire, toutes les variantes.

## Revendications

1. Procédé d'oxydation d'aldoses et notamment du glucose en acides aldoniques correspondants, caractérisé par le fait que l'oxydation est réalisée en milieu alcalin au moyen d'un gaz contenant de l'oxygène en ayant recours à un catalyseur qui a été obtenue par apport ultérieur d'une quantité efficace d'au moins un des métaux du groupe de ceux constituant les classes IV, V et VI, ou promoteur, sur un catalyseur constitué par du palladium sur support inerte et ui est employé tel quel.

2. Procédé selon la revendication 1, caractérisé par le fait que le promoteur est choisi parmi le bismuth, le plomb, l'antimoine, l'étain ou le sélénium, avec une préférence toute particulière pour le bismith et le plomb.

3. Procédé selon l'une des revendications 1 ou 2, caractérisé par le fait que le support inerte est choisi parmi le charbon finement divisé, l'alumine, la silice, la silice-alumine, le sulfate de baryum et l'oxyde de titane.

4. Procédé selon l'une des revendications 1 à 3, caractérisé par le fait que la teneur du catalyseur en palladium exprimée en tant que métal est comprise entre 1 et 10% en poids par rapport au support inerte.

5. Procédé selon l'une des revendications 1 à 4, caractérisé par le fait que la teneur du catalyseur en promoteur, notamment en bismuth et/ou en plomb, exprimée en tant que métal, est comprise entre 1 et 300% et, de préférence, entre 5 et 100% en poids par rapport au palladium.

6. Procédé selon l'une des revendications 1 à 5, caractérisé par le fait qu'il est appliqué à l'oxydation d'aldoses du groupe comprenant l'érythrose, le thréose, le ribose, l'arabinose, le xylose, le lyxose, l'allose, l'altrose, le gulose, le mannose, l'idose, le galactose, le talose et leur mélange.

7. Procédé selon l'une des revendications 1 à 6, caractérisé par le fait ques les aldoses sont mis en oeuvre sous la forme d'une solution aqueuse d'une concentration comprise entre 5 et 60% en poids.

8. Procédé selon l'une des revendications 1 à 6, caractérisé par le fait que, pour l'oxydation du glucose en gluconate de sodium, on met en oeuvre une solution de glucose d'une concentration comprise entre 20 et 40% en poids.

9. Procédé selon l'une des revendications 1 à 8, caractérisé par le fait que le catalyseur est dispersé dans la solution d'aldoses en une quantité telle que la concentration en palladium, exprimée en tant que métal, soit comprise entre 0,005 et 1% en poids par rapport aux aldoses et de préférence ente 0,01 et 0,4%.

10. Procédé selon l'une des revendications 1 à 9, caractérisé par le fait que la température à laquelle est conduite la réaction d'oxydation est comprise entre 20 et 90°C, de préférence entre 25 et 60°C, pour un temps de réaction compris entre 30 minutes et 5 heures.

11. Procédé selon l'une des revendications 1 à 10, caractérisé par le fait que le pH du milieu réactionnel est maintenu à l'aide d'un ou plusieurs agents alcalins à une valeur comprise entre 7,5 et 11,0, de préférence entre 8,0 et 10,0.

12. Procédé selon l'une des revendications 1 à 11, caractérisé par le fait que l'agent alcalin est choisi dans le groupe comprenant l'hydroxyde de calcium, l'hydroxyde de lithium, l'hydroxyde de magnésium, les carbonates de zinc ou de manganése, ou tous autres sels de zinc ou de manganèse pour lesquels les hydroxydes correspondants sont obtenus in situ par addition d'un agent alcalin tel que l'hydroxyde de sodium ou l'hydroxyde de potassium.

13. Application a la preparation d'acides aldoniques par oxydation d'aldoses, d'un catalyseur à base de palladium déposé sur support inerte, ledit catalyseur étant "dopé" ultérieurement à l'aide d'un ou plusieurs des métaux ou promoteurs des groupes IV, V ou VI de la classification périodique, ledit promoteur étant de préférence choisi parmi le bismuth et le plomb, l'antimoine, l'étain et le sélénium, le support inerte étant de préférence choisi parmi le charbon finement divisé, l'alumine, la silice, la silice-alumine, le sulfate de baryum et l'oxyde de titane.

## Patentansprüche

1. Verfahren zur Oxidation von Aldosen, insbesondere von Glucose, zu den entsprechenden Aldonsäuren, dadurch gekennzeichnet, daß die Oxidation in einem alkalischen Medium mit Hilfe eines sauerstoffhältigen Gases und unter Verwendung eines Katalysators durchgeführt wird, welcher Katalysator durch weitere Zufuhr einer wirksamen Menge von wenigstens einem Metall, oder Promotor, aus der Gruppe jener, welche die gruppen IV, V und VI darstellen, zu einem Katalysator, welcher aus Palladium auf einem inerten Träger besteht, erhalten worden ist, und der so, wie er erhalten wird, eingesetzt wird.

2. Verfahren nach Anspruch 1, dadurch gekennzeichnet, daß der Promotor aus Wismut, Blei, Antimon, Zinn oder Selen ausgewählt wird, wobei Wismut und Blei ganz besonders bevorzugt sind.

3. Verfahren nach Anspruch 1 oder 2, dadurch gekennzeichnet, daß der inerte Träger aus fein verteilter Kohle, Aluminiumoxid, Kieselsäureanhydrid, Kieselsäureanhydrid-Aluminiumoxid, Bariumsulfate und Titanoxid ausgewählt ist.

4. Verfahren nach einem der Ansprüche 1 bis 3, dadurch gekennzeichnet, daß der Gehalt des Katalysators an Palladium, als Metall ausgedrückt, zwischen 1 und 10 Gew.-%, bezogen auf den inerten Träger, beträgt.

5. Verfahren nach einem der Ansprüche 1 bis 4, dadurch gekennzeichnet, daß der Gehalt des Katalysators an dem Promotor, insbesondere an Wismut und/oder Blei, ausgedrückt als Metall, zwischen 5 und 100 Gew.-% bezogen auf das Palladium, beträgt.

6. Verfahren nach einem der Ansprüche 1 bis 5, dadurch gekennzeichnet, daß es für die Oxidation von Aldosen angewendet wird, welche Aldosen aus der Erythrose, Threose, Ribose, Arabinose, Xylose, Lyxose, Allose, Altrose, Gulose, Mannose, Idose, Galactose, Talose und deren Gemischen umfassenden Gruppe ausgewählt sind.

7. Verfahren nach einem der Ansprüche 1 bis 6, dadurch gekennzeichnet, daß die Aldosen in der Form einer wässerigen Lösung mit einer zwischen 5 und 60 Gew.-% liegenden Konzentration eingesetzt werden.

8. Verfahren nach einem der Ansprüche 1 bis 6, dadurch gekennzeichnet, daß man für die Oxidation von Glucose zu Natriumgluconat eine Glucoselösung mit einer zwischen 20 und 40 Gew.-% liegenden Konzentration einsetzt.

9. Verfahren nach einem der Ansprüche 1 bis 8, dadurch gekennzeichnet, daß der Katalysator in der Aldosenlösung in einer solchen Menge dispergiert ist, daß die Konzentration an Palladium, als Metall ausgedrückt, zwischen 0,005 und 1 Gew.-%, vorzugsweise zwischen 0,01 und 0,4 Gew.-%, bezogen auf die Aldosen, beträgt.

10. Verfahren nach einem der Ansprüche 1 bis 9, dadurch gekennzeichnet, daß die Temperatur, bei welcher die Oxidationsreaktion durchgeführt wird, zwischen 20 und 90°C, vorzugsweise zwischen 25 und 60°C, liegt, und daß die Reaktionszeit zwischen 30 min und 5 h beträgt.

11. Verfahren nach einem der Ansprüche 1 bis 10, dadurch gekennzeichnet, daß der pH-Wert des Reaktionsmediums mit Hilfe eines oder mehrerer alkalischer Mittel auf einem zwischen 7,5 und 11,0, vorzugsweise ziwchen 8,0 und 10,0 liegenden Wert gehalten wird.

12. Verfahren nach einem der Ansprüche 1 bis 11, dadurch gekennzeichnet, daß das alkalische Mittel aus einer Calciumhydroxid, Lithiumhydroxid, Magnesiumhydroxid, die Carbonate von Zink oder Mangan, oder jedwede andere Salze von Zink oder Mangan, für welche die entsprechenden Hydroxide in stiu durch Zugabe eines alkalischen Mittels, wie z.B. von Natriumhydroxid oder Kaliumhydroxid, erhalten werden, umfassenden Gruppe ausgewählt wird.

13. Verwendung eines Katalysators auf der Basis von Palladium, welches auf einem inerten Träger aufgebracht ist, für die Herstellung von Aldonsäuren durch Oxidation von Aldosen, wobei dieser Katalysator weiterhin mit Hilfe von einem oder mehreren Metallen oder Promotoren aus den Gruppen IV, V oder VI des Periodensystems "dopiert" worden ist, wobei dieser Promotor vorzugsweise aus Wismut und Blei, Antimon, Zinn und Selen ausgewählt ist, und wobei er inerte Träger vorzugsweise aus fein verteilter Kohle, Aluminiumoxid, Kieselsäureanhydrid, Kieselsäureanhydrid-Aluminiumoxid, Bariumsulfat und Titanoxid ausgewählt ist.

## Claims

1. Process for the oxidation of aldoses and especially of glucose into the corresponding aldonic acids, characterized by the fact that the oxidation is carried out in an alkaline medium by means of an oxygen-containing gas, in the presence of a catalyst obtained by further addition an efficient quantity of at least one of the metals of the group constituted by the Groups IV, V and VI, or promoter onto a catalyst consisting of palladium on an inert support and which is used as such.

2. Process according to claim 1, characterized by the fact that the promoter is selected from bismuth, lead antimony, tin or selenium, with a most particular preference for bismuth and lead.

3. Process according to one of claims 1 or 2, characterized by the fact that the inert support is selected from finely divided carbon, alumina, silica, silica-alumina, barium sulfate and titanium oxide.

4. Process according to one of claims 1 to 3, characterized by the fact that the palladium content of the catalyst, expressed in terms of metal, is comprised between 1 and 10% with respect to the inert support.

5. Process according to one of claims 1 to 4, characterized by the fact that the promoter content of the catalyst, especially the bismuth and/or lead content, expressed in terms of metal, is comprised between 1 and 300% and, preferably, between 5 and 100 wt% with respect to palladium.

6. Process according to one of claims 1 to 5, characterized by the fact that it is used for the oxidation of aldoses of the group comprising erythrose, threose, ribose, arabinose, xylose, lyxose, allose, altrose, gulose, mannose, idose, galactose, talose and a mixture thereof.

7. Process according to one of claims 1 to 6, characterized by the fact that the aldoses are used in the form of an aqueous solution having a concentration comprised between 5 and 60 wt%.

8. Process according to one of claims 1 to 6, characterized by the fact that, in order to oxidize the glucose into sodium gluconate, a glucose solution having a concentration comprised between 20 and 40 wt% is used.

9. Process according to one of claims 1 to 8, characterized by the fact that the catalyst is dispersed in the solution of aldoses in a quantity such that the palladium concentration, expressed in terms of metal, is comprised between 0.005 and 1 wt% with respect to the aldoses and preferably between 0.01 and 0.4%.

10. Process according to one of claims 1 to 9, characterized by the fact that the temperature at which the reaction of oxidation is carried out, ranges between 20 and 90°C, preferably between 25 and 60°C, for a reaction time comprised between 30 minutes and 5 hours.

11. Process according to one of claims 1 to 10, characterized by the fact that the pH of the reaction medium is maintained by means of one or several alkaline agents at a value comprised between 7.5 and 11.0, preferably between 8.0 and 10.0.

12. Process according to one of claims 1 to 11, characterized by the fact that the alkaline agent is selected from the group comprising calcium hydroxide, lithium hydroxide, magnesium hydroxide, zinc or manganese carbonates, or any other zinc or manganese salts for which the corresponding hydroxides are obtained in stiu by adding an alkaline agent such as sodium hydroxide or potassium hydroxide.

13. Use for the preparation of aldonic acids by oxidation of aldoses, of a palladium-based catalyst deposited onto an inert support, said catalyst being subsequently "doped" with one or several of the metals or promoters from Groups IV, V or VI of the Period System, said promoter being preferably selected from bismuth and lead, antimony, tin an selenium, the inert support being preferably selected from finely divided carbon, alumina, silica, silica-alumina, barium sulfate and titanium oxide.